# EUROPÄISCHE PATENTSCHRIFT

(11) **EP 1 807 699 B1**
(45) Veröffentlichungstag und Bekanntmachung des Hinweises auf die Patenterteilung: **31.12.2008**
(21) Anmeldenummer: 05802857.2
(22) Anmeldetag: 04.11.2005
(51) Int. Cl.: G01N 33/543, H01J 49/04

(54) **STRUKTURIERTE COPOLYMERE TRÄGER FÜR DIE SPEKTROMETRIE ODER SPEKTROSKOPIE**
STRUCTURED COPOLYMER SUPPORTS FOR USE IN SPECTROMETRY OR SPECTROSCOPY
SUPPORTS COPOLYMÈRES STRUCTURÉS DESTINÉS À LA SPECTROMÉTRIE OU À LA SPECTROSCOPIE

(30) Priorität: 05.11.2004 DE 102004053458
(43) Veröffentlichungstag der Anmeldung: 18.07.2007
(73) Patentinhaber: MPG Max-Planck-Gesellschaft zur Förderung der Wissenschaften e.V., 80539 München (DE)
(72) Erfinder: SVATOS, Ales, 07745 Jena (DE); MUCK, Alexander, 07743 Jena (DE)
(74) Vertreter: Katzameyer, Michael
(86) Internationale Anmeldenummer: PCT/EP2005/011834
(87) Internationale Veröffentlichungsnummer: WO 2006/048306

(56) Entgegenhaltungen:
- WO-A-20/04024318
- US-A1- 2003 207 460
- US-A1- 2004 084 615
- US-A1- 2004 094 705
- PETERSON DOMINIC S ET AL: "Porous polymer monolith for surface-enhanced laser desorption/ionization time-of-flight mass spectrometry of small molecules." RAPID COMMUNICATIONS IN MASS SPECTROMETRY : RCM. 2004, Bd. 18, Nr. 13, 15. Juli 2004 (2004-07-15), Seiten 1504-1512, XP002362030 ISSN: 0951-4198
- XU YINGDA ET AL: "Non-specific, on-probe cleanup methods for MALDI-MS samples" MASS SPECTROM REV; MASS SPECTROMETRY REVIEWS NOVEMBER/DECEMBER 2003, Bd. 22, Nr. 6, November 2003 (2003-11), Seiten 429-440, XP002362031
- KAI J. ET AL: "Protein microarray on cyclic olefin copolymer (COC) for disposable protein lab-on-a-chip" 7TH INTERNATIONAL CONFERENCE ON MINIATURIZED CHEMICAL AND BIOCHEMICAL ANALYSIS SYSTEMS, 5. Oktober 2003 (2003-10-05), Seiten 1101-1104, XP002362032 ISSN: 1546-198X
- FRANKEVICH VLADIMIR E ET AL: "Role of electrons in laser desorption/ionization mass spectrometry." ANALYTICAL CHEMISTRY. 15 NOV 2003, Bd. 75, Nr. 22, 15. November 2003 (2003-11-15), Seiten 6063-6067, XP002362033 ISSN: 0003-2700
- MARKO-VARGA G ET AL: "Disposable polymeric high-density nanovial arrays for matrix assisted laser desorption/ionization-time of flight-mass spectrometry: I. Microstructure development and manufacturing." ELECTROPHORESIS. OCT 2001, Bd. 22, Nr. 18, Oktober 2001 (2001-10), Seiten 3978-3983, XP002362034 ISSN: 0173-0835 in der Anmeldung erwähnt
- TIMOFEEV E ET AL: "Binding specificity and stability of duplexes formed by modified oligonucleotides with a 4096-hexanucleotide microarray" NUCLEIC ACIDS RESEARCH, OXFORD UNIVERSITY PRESS, SURREY, GB, Bd. 29, Nr. 12, Juni 2001 (2001-06), Seiten 2626-2634, XP002961131 ISSN: 0305-1048
- MUCK ALEXANDER ET AL: "Fast prototyping of hydrophobic disposable polymer support arrays for matrix-assisted laser desorption/ionization-time of flight-mass spectrometry of proteins by atmospheric molding" ELECTROPHORESIS, Bd. 26, Nr. 14, Juli 2005 (2005-07), Seiten 2835-2842, XP002362035 ISSN: 0173-0835

## Beschreibung

Die vorliegende Erfindung betrifft neue, strukturierte, copolymere Probenträger-Chips, insbesondere für die Massenspektrometrie, und deren Verwendung.

In den letzten Jahren gewannen massenspektrometrische Techniken als Verfahren zur Analyse von biologischen Makromolekülen wie Proteinen und Nukleinsäuren zunehmend an Bedeutung. Insbesondere die matrix-unterstützte Laserdesorptions/Ionisations-Massenspektrometrie (MALDI-MS) und oberflächen-unterstützte Laserdesorptions/Ionisations-Massenspektrometrie (SELDI-MS) sind grundsätzlich effiziente und in letzter Zeit häufig eingesetzte Verfahren zur Bestimmung der Molekülmassen von Biomolekülen wie Proteinen. Trotzdem gibt es bei ihrer Anwendung zur Analyse mancher Proteine wie z.B. Membranproteine oder mit Salz verunreinigter Proteinproben nach wie vor Probleme und die Empfindlichkeit der Assays ist manchmal unzureichend.

Als Trägeroberflächen für die Proteinproben wurden bereits unterschiedlichste Materialien, z.B. Metalle, beschichtete Metalle und polymere Kunststoffe, eingesetzt. In jüngster Zeit wurden aufgrund ihrer preisgünstigen Herstellung und guten Signalausbeute von verschiedenen Autoren polymere Kunststoffträger empfohlen. Als geeignete Beispiele für solche polymeren Kunststoffe wurden im Stand der Technik z.B. Polyethylenmembranen (Worrall et al., Anal. Chem. 1998, 70, 750-756) und Träger auf Basis von Poly(methylmethacrylat) (PMMA) und Polycarbonat (PC) (Marko-Varga et al.., Elektrophoresis, 2001 Bd. 22, 3978-3983; Ekström et al. Elektrophoresis 2001, Bd. 22, 3984-3992) beschrieben.

Nachteilig bei diesen Trägern des Standes der Technik ist jedoch, dass sie aus handelsüblichen Polymeren und vorgegossenen Kunstoffolien hergestellt wurden, so dass die Oberflächeneigenschaften des polymeren Trägermaterials vorgegeben waren. Darüber hinaus wurden zur Herstellung dieser Träger relativ teure und aufwendige herkömmliche Verfahren eingesetzt, welche aus der Polymer- und Elektronikindustrie entlehnt waren.

Die Druckschrift US 2003/0207460 offenbart Copolymere, die photoreaktive Gruppierungen von energiabsorbierenden Molekülen (EAM) enthalten, zur Verwendung bei der Desorptions/Ionisations-Massenspektrometrie. Diese Copolymere werden als Überzug auf einen herkömmlichen Glasträger aufgebracht und unterstützen die Desorption/Ionisation der Proben aktiv, indem sie Photostrahlung absorbieren, um thermische Energie zur erzeugen und die thermische Energie übertragen.

Peterson et al. Rapid Commun. Mass Spectrom. 2004, Bd. 18, 1504-1512 offenbart poröse Poly(butylmethacrylat-co-ethylen-dimethacrylat) sowie Poly(benzylmethacrylat-co-ethylen-dimethacrylat) Monolithe, die auf normalen MALDI-Massen-Spektrometrie Probenträger-Chips hergestellt werden.

Eine erste Aufgabe der vorliegenden Erfindung gegenüber dem Stand der Technik bestand in der Erhöhung der Nachweisempfindlichkeit von massenspektrometrischen Assays für biologische Makromoleküle wie Proteine und Nukleinsäuren, bei denen solche polymeren Probenträger verwendet werden, auf einfache und kostengünstige Weise.

Eine damit in Zusammenhang stehende zweite Aufgabe bestand in der Bereitstellung eines strukturierten copolymeren Probenträger-Chips für die Spektroskopie und Spektrometrie, insbesondere die Massenspektrometrie, mit verbesserten Oberflächeneigenschaften, die in Abhängigkeit von dem speziell zu untersuchenden Analyten eingestellt werden können, um z.B. die Reinigung/Entsalzung des Analyten direkt auf dem Träger zu ermöglichen.

Hierzu ergaben eingehende Untersuchungen der Erfinden, dass durch eine gezielte Auswahl bzw. Modifikation des polymeren Trägermaterials, um bestimmte Oberflächeneigenschaften, z.B. Hydrophobizität, Polarität etc., zu erzeugen oder zu fördern, die Nachweisempfindlichkeit eines massenspektrometrischen Assays zur Analyse von Biomolekülen, wie z.B. Proteinen, Peptiden und Nukleinsäuren, bei dem diese modifizierten Träger verwendet werden, erheblich gesteigert werden konnte. Bei den Probenträgern des Standes der Technik kann eine solche Modifikation nur durch eine nachträgliche Derivati- sierung der Oberfläche durch eine chemische und/oder physikalische Behandlung erfolgen. Eine solche Behandlung ist zeitaufwendig und führt oft zu nicht optimalen Ausbeuten an gewünschtem Produkt.

Die Erfinder stellten nun fest, dass strukturierte copolymere Probenträger-Chips mit gewünschten Oberflächeneigenschaften auf einfache, schnelle und kostengünstige Weise hergestellt werden können, indem eine Polymerisationslösung, welche die benötigten Monomerkomponenten zur Erzielung eines Polymers bzw. Copolymers mit den gewünschten Eigenschaften enthält, direkt in einer Form, welche der vorgesehenen dreidimensionalen Struktur des Träger-Chips entspricht, zur Polymerisation gebracht wird.

Aufgrund der Einfachheit und Schnelligkeit dieses Verfahren können die Probenträger-Chips auch in kleinen Labors für z.B. massenspektrometrische Analysen maßgeschneidert für die besonderen Anforderungen der jeweiligen Proben hergestellt werden, um auf diese Weise die Nachweisempfindlichkeit von massenspektrometrischen Assays zu optimieren.

Die obengenannten Aufgaben werden somit erfindungsgemäß gelöst durch die Bereitstellung strukturierter copolymerer Probenträger-Chips nach den Ansprüchen 1-6 und deren Verwendung in der Spektroskopie oder Spektrometrie, insbesondere Massenspektrometrie, nach den Ansprüchen 7-12.

### Beschreibung der Erfindung

Die vorliegende Erfindung betrifft neue strukturierte copolymere Probenträger-Chips für die Spektrometrie und Spektroskopie, insbesondere Probenträger-Chips für die Massenspektrometrie, mit verbesserten Oberflächeneigenschaften und offenbart ein besonders vorteilhaftes Verfahren zur Herstellung dieser strukturierten copolymeren Probenträger-Chips, bei dem eine Polymerisierungslösung, welche die zu polymerisierenden Monomere oder Makromonomere enthält, in einer Form, welche ein Negativ der gewünschten Struktur umfasst, zur Polymerisation gebracht wird und die gebildeten Polymerisate aus der Form gelöst werden.

Der Begriff "Polymerisation", wie hier gebraucht, soll alle Arten der Überführung von relativ niedermolekularen Verbindungen, "Monomeren", in hochmolekulare Verbindungen, "Polymere", umfassen und neben der Polymerisation im engeren Sinne, die stufenlos verläuft, auch die Polykondensation und Polyaddition als Stufenreaktionen umfassen. Die relativ niedermolekularen Verbindungen können auch Oligomere oder kurzkettige Polymere (Molekülmasse im Bereich von etwa 100 bis 100000 Dalton) sein, so genannte "Makromonomere", die wie einfache Monomere weiter polymerisiert werden. Damit können z.B. Pfropfcopolymere hergestellt werden, bei denen das Hauptpolymergerüst mit Seitenketten eines bestimmten Comonomers versehen ist.

Dementsprechend können die hergestellten copolymeren TrägerChips innerhalb der Maßgaben von Anspruch 1 aus einem breiten Spektrum bekannter Kunststoffe, z.B. copolymere Derivate von Polyestern, Polycarbonaten, Polyolefinen, Poly(meth)acrylaten, insbesondere Polymethylmethacrylaten ausgewählt sein, mit der Maßgabe, dass die grundsätzliche Eignung für den gewünschten Verwendungszweck, z.B. als Probenträger-Chips für die Massenspektrometrie, gegeben sein muß. Die jeweils erforderlichen Grundeigenschaften, z.B. Härte, Beständigkeit gegenüber Lasereinwirkung, bestimmten Lösungsmitteln etc., und die entsprechenden grundsätzlich geeigneten Kunststoffe sind für den Fachmann unschwer ersichtlich.

Erfindungsgemäß werden die Oberflächeneigenschaften des copolymeren Träger-Chips durch geeignete Wahl der Monomerkomponenten in gewünschter Weise bestimmt wobei es sich bei einem Monomer um Methylmethacrylat handelt. Beispielsweise können durch Verwendung eines Monomers mit stark polaren oder geladenen Substituenten die Oberflächenladung beeinflußt und ionische Wechselwirkungen gefördert werden. Zur Erzeugung oder Erhöhung einer negativen Oberflächenladung und Anziehung von positiv geladenen Analyten können z.B. Monomere, bei denen es sich um ein substituiertes Alkylmethacrylat, das eine Sulfo-, Hydroxy- oder Carboxygruppe aufweist, eingesetzt werden. Einige nicht-beschränkende Beispiele hierfür sind Carboxyethylacrylat, Hydroxymethylmethacrylat, Hydroxyethylmethacrylat, Hydroxypropylmethacrylat, Acryloyloxyhydroxypropylmethacrylat, 2-(2-Ethoxyethoxy)ethylmethacrylat, Sulfoethylmethacrylat, Sulfopropylacrylat, etc. Zur Erzeugung oder Erhöhung einer positiven Oberflächenladung und Anziehung von negativ geladenen Analyten können z.B. Monomere, bei denen es sich um ein substituiertes Alkylmethacrylat, das eine Aminogruppe aufweist, verwendet werden. Einige nicht-beschränkende Beispiele hierfür sind Aminoethylmethacrylat, Aminopropylmethacrylat, N-(3-Aminopropyl)methacrylamid etc. das Basismonomer Methylmethacrylat mit einer mäßigen Hydrophobizität kann durch Copolymerisation mit einem stärker hydrophoben Monomer in ein Copolymer mit einem gewünschten Grad an Hydrophobizität überführt werden, um damit z.B. die Bindung von hydrophoben Proben an die Trägerchipoberfläche zu verstärken. Einige geeignete, nicht-beschränkende Beispiele für hydrophobere Comonomere sind C₂₋₁₈-Alkylmethacrylat, Hexafluorbutylmethacrylat und andere fluorierte Alkylmethacrylate. Durch die Verwendung eines teilweise aromatischen Comonomers kann die Wechselwirkung des nicht-aromatischen Basishomopolymers mit Analyten, die eine aromatische oder heteroaromatische Gruppe enthalten, innerhalb der Maßgaben von Anspruch 1 verstärkt werden. Solche Analyten sind z.B. Proteine und Peptide, die Phenylalanin, Tryptophan oder Tyrosin enthalten, aromatische Fettsäuren und Triglyceride. Einige geeignete, nicht-beschränkende Beispiele für aromatische Comonomere sind Benzylacrylat, Benzylmethacrylat, Divinylbenzol, Styrol und Derivate davon, Furfurylmethacrylat, Anthracenylmethylacrylat, N-Acryloxysuccinimid, 4-Chlorphenylacrylat, 4-Methacryloxy-2-hydroxybenzophenon, 2-(2'-Methacryloxy-5'-methylphenyl)benzotriazol etc.

In einer bevorzugten Ausführungsform des Verfahrens finder eine stufenlose Polymerisation statt und die verwendete Polymerisierungslösung umfasst innerhalb der Maßgaben von Anspruch 1 mindestens zwei Monomere oder davon abgeleitete Makromonomere, die jeweils mindestens eine Vinylgruppe umfassen.

Vorzugsweise sind die Comonomere des Kauptmonomers Methylmethacrylat aus der Gruppe aus substituierten (Meth)acrylaten, (Meth)acrylamiden, substituierten (Meth)acrylamiden, (Meth)acrylnitril, substituiertem (Meth)acrylnitril, Styrol und substituierten Styrolen, Divinylbenzol und substituiertem Divinylbenzol, Butadien, Ethylenglycoldimethacrylat, Di(ethylenglycol)dimethacrylat, Ethylenglycoldiacrylat, Di(ethylenglycol)diacrylat, 3-(Acryloyloxy)-2-hydroxypropylmethacrylat und N,N'-Methylenbismethacrylamid ausgewählt.

Spezieller handelt es sich innerhalb der Maßgaben von Anspruch 1 bei den substituierten (Meth)acrylaten um Alkyl(meth)acrylate, substituierte Alkyl(meth)acrylate, Aryl(meth)acrylate oder substituierte Aryl(meth)acrylate. Die substituierten Alkyl(meth)acrylate oder substituierten Aryl(meth)acrylate können z.B. Sulfo-, Hydroxy-, Carboxy- oder Aminofunktionalitäten aufweisen. Einige nicht-beschränkende Beispiele hierfür sind Carboxyethylacrylat, Hydroxymethylmethacrylat, Hydroxyethylmethacrylat, Hydroxypropylmethacrylat, Acryloyloxyhydroxypropylmethacrylat, 2-(2-Ethoxyethoxy)ethylmethacrylat, Sulfoethylmethacrylat, Sulfopropylacrylat , Aminoethylmethacrylat, Aminopropylacrylat etc.

Die Polymerisierungsmischung umfasst als Hauptmonomer Methylmethacrylat und ein oder mehrere Comonomere, welche(s) die Oberflächeneigenschaften des Polymethylmethacrylat-Homopolymers in gewünschter Weise modifiziert/en.

Eine Möglichkeit zur Erhöhung der Hydrophobizität ist beispielsweise die Inkorporation eines Alkylmethacrylat-Comonomers mit einer längeren Alkylkette als Methyl oder Ethyl, vorzugsweise im Bereich von C₃-C₁₈, z.B. Propyl, Butyl, Pentyl, Hexyl, Heptyl, Octyl, Nonyl, Decyl, Undecyl, Duodecyl bis Octadecyl, oder von fluorierten Alkylmethacrylaten wie oben genannt.

Andererseits kann durch Verwendung eines Comonomers mit stark polaren oder geladenen Substituenten die Oberflächenladung beeinflusst und die ionische Wechselwirkung mit bestimmten Proben gefördert werden.

Zur Erzeugung oder Erhöhung einer negativen Oberflächenladung und Anziehung von positiv geladenen Analyten können beispielsweise substituierte Alkylmethacrylate mit sulfo-, Hydroxy- oder Carboxygruppen als Comonomere eingesetzt werden. Einige nicht beschränkende Beispiele sind Carboxyethylacrylat, Hydroxymethylmethacrylat, Hydroxyethylmethacrylat, Hydroxypropylmethacrylat, Acryloyloxyhydroxypropylmethacrylat, 2-(2-Ethoxy-ethoxy)ethylmethacrylat, Sulfoethylmethacrylat, Sulfopropylacrylat.

Zur Erzeugung oder Erhöhung einer positiven Oberflächenladung und Anziehung von negativ geladenen Analyten können als Comonomere substituierte Alkylmethacrylate mit Aminogruppen, vorzugsweise Aminoalkylmethacrylate, z.B. Aminoethylmethacrylat, Aminopropylmethacrylat, verwendet werden. Zur Erhöhung der n-n-Wechselwirkungen mit Analyten, die eine aromatische oder heteroaromatische Gruppe enthalten, können innerhalb der Maßgaben von Anspruch 1 Comonomere mit Arylgruppen, vozugsweise Arylmethacrylate, eingesetzt werden. Einige nicht beschränkende Beispiele hierfür sind Benzylacrylat, Benzylmethacrylat, Anthracenylmethylacrylat, Furfurylmethacrylat, 4-Chlorphenylacrylat.

Die Anteile der verschiedenen Comonomere können je nach Bedarf von 0,001 bis 99,999 %, spezieller von 0,01 bis 99,99 %, typischerweise von 0,1 bis 99,9 %, vorzugsweise von 1 bis 99 %, besonders bevorzugt 10 bis 90 %, variieren.

In einem anderen Aspekt, der kein Teil der vorliegenden Erfindung ist enthält die Polymerisierungslösung im wesentlichen nur ein Monomer, so dass ein Homopolymer gebildet wird. Ein bevorzugtes Monomer in dieser Ausführungsform ist ein C₄₋₆-Alkylmethacrylat, vorzugsweise Butylmethacrylat oder ein substituiertes, z.B. hydrophil substituiertes, Derivat davon.

Die eingesetzte Polymerisationsreaktion kann eine ionische oder radikalische Polymerisation sein. Dementsprechend kann die Polymerisierungslösung neben den Monomeren sowie gegebenenfalls Lösungsmitteln und/oder anderen Hilfsstoffen auch einen Katalysator für die ionische oder radikalische Polymerisation enthalten. Geeignete Katalysatoren für den jeweiligen Reaktionstyp und die jeweiligen Monomertypen sind im Stand der Technik bekannt.

Vorzugsweise ist die Polymerisation eine Radikalpolymerisation, welche z.B. durch Röntgen- oder Gammastrahlung, UV-Licht oder durch thermische Behandlung initiiert werden kann, und die Polymerisierungslösung enthält einen Radikalpolymerisationskatalysator. Insbesondere für Copolymere auf Methacrylatbasis ist Benzoinmethylether (α-Methoxy-α-phenylacetophenon) ein bevorzugter Katalysator für eine Radikalpolymerisation unter UV-Licht. Andere bekannte Katalysatoren sind jedoch ebenfalls geeignet.

In einer stark bevorzugten Ausführungsform wird die Radikalpolymerisation durch UV-Licht initiiert. Insbesondere für die Polymerisation von Monomeren auf Methacrylatbasis wird dabei vorzugsweise UV-Licht einer Wellenlänge im Bereich von 350-400 nm, insbesondere etwa 365 nm, eingesetzt.

Die Polymerisation und das Formen der strukturierten Träger-Chips kann grundsätzlich unter verschiedenen Bedingungen nach jedem bekannten und geeigneten Verfahren, z.B. Spritzguss, Pressguss, Formen unter Atmosphärendruck etc., erfolgen.

Besonders vorteilhaft ist jedoch, dass die Herstellung der strukturierten Träger-Chips unter Umgebungsbedingungen, d.h. Atmosphärendruck und Umgebungstemperatur, erfolgt. Auf diese Weise sind keine aufwendigen und teuren Geräte erforderlich und die Erfinder haben festgestellt, dass die Polymerisationsreaktion unter diesen Bedingungen trotzdem schnell und vollständig ablaufen kann. Für Polymere bzw. Copolymere auf Methacrylatbasis ist die UV-induzierte Polymerisationsreaktion in der Regel bereits nach wenigen Stunden, vorzugsweise etwa 1 bis 2 Stunden, abgeschlossen.

Das Formen der polymeren Träger-Chips findet vorzugsweise gleichzeitig mit der Polymerisation statt, indem eine Polymerisierungslösung, welche die zu polymerisierenden Monomere oder Makromonomere enthält, direkt in einer Form, welche ein Negativ der gewünschten Struktur umfasst, zur Polymerisation gebracht wird. Es ist jedoch auch möglich, zuerst ein unstrukturiertes Polymerisat herzustellen und diesem in einem zweiten Schritt nach irgendeinem geeigneten Verfahren, z.B. Spritzguss oder Prägung, die gewünschte Struktur zu geben.

Das Formteil, welches das Negativ der gewünschen Trägerchipstruktur repräsentiert, kann grundsätzlich aus verschiedenstem Material, z.B. Glas, Metall, Kunststoff, bestehen, je nach bedarf sehr unterschiedliche Größe und Form aufweisen und auf jede bekannte Weise hergestellt werden. Es ist jedoch erforderlich, dass die gewünschten Mikrostrukturen, die bei Träger-Chips für die Massenspektrometrie typischerweise eine Tiefe im Bereich von 1 bis 1000 µm, häufiger 10 bis 100 µm, z.B. etwa 50 µm, aufweisen, mit großer Genauigkeit auf die Polymerisationsprodukte übertragen und die resultierenden Polymerisate bzw. Copolymerisate unschwer aus der Form gelöst werden können. Darüber hinaus sollte das Formteil möglichst einfach und kostengünstig herstellbar sein.

In einer stark bevorzugten Ausführungsform ist dieses Formteil ein Silicium-Wafer und besonders bevorzugt wird die Struktur des Silicium-Wafers durch Photolithographie, gefolgt von einer chemischen und/oder physikalischen Behandlung des Wafers, z.B. nassem chemischem Ätzen, erzeugt. Ein derartiges Verfahren zur Herstellung von Silicium-Wafern als Negativ für strukturierte Mikrochips auf PMMA-Basis wurde von den Erfindern der vorliegenden Erfindung bereits beschrieben (Anal. Chem. 2004, 76, 2290-2297) und für die erfindungsgemäße Herstellung strukturierter copolymerer Probenträger-Chips für die Massenspektrometrie adaptiert und weiterentwickelt. Die Herstellung eines entsprechenden Silicium-Wafers nach diesem einfachen und effizienten Verfahren wird in Beispiel 1 detailliert beschrieben. Die Bildung einer geeigneten Form unter Verwendung dieses Silicium-Wafers dauert nur wenige Minuten und wird in Beispiel 2 ebenso beschrieben wie die eigentliche Polymerisation und nachfolgende Weiterbehandlung der gebildeten Polymerisate.

Die erfindungsgemäßen copolymeren Probenträger-Chips besitzen gegenüber herkömmlichen Probenträgern verbesserte Oberflächeneigenschaften, die in Abhängigkeit von den speziell zu untersuchenden Analyten eingestellt werden können. Die Analyten sind vorzugsweise Biomoleküle, d.h. in lebenden Organismen natürlicherweise vorkommende Verbindungen, und Metabolite, also Stoffwechselprodukte unterschiedlicher Art. Die Analyten sind typischerweise Proteine, Peptide, Nukleinsäuren, Lipide und andere kleine und große Biomoleküle, können jedoch auch kleine und große Moleküle nicht-biologischen Ursprungs umfassen. Ein besonderer Vorteil dieser Träger-Chips besteht darin, dass das Probenmaterial Verunreinigungen wie Salze, Detergenzien, Puffer etc. enthalten kann, die sich aufgrund der schlechteren Bindung an die Trägeroberfläche unschwer von dem stärker bindenden Analyten abtrennen lassen. Das von Verunreinigungen befreite Analyt-Molekül liefert dann direkt in dem jeweiligen Nachweissystem, vorzugsweise MALDI- oder SELDI-Massenspektrometrie und/oder Emissionspektroskopie oder Absorptionsspektroskopie (wie z.B. die Fluoreszenzspektroskopie), ein vergleichsweise stärkeres Signal mit geringerem Hintergrundrauschen und kann in geringeren Konzentrationen nachgewiesen werden. Das heißt die Nachweisempfindlichkeit solcher Assays kann erheblich gesteigert werden.

Die erfindungsgemäßen Probenträger-Chips ermöglichen somit ohne weitere Modifikationschritte die Probenreinigung oder spezifische Adsorption der Proben und deren direkte Messung auf der gleichen Plattform. Im Gegensatz zu bekannten Systemen des Standes der Technik sind keine Pumpensysteme und längeren Separationszeiten erforderlich, das Anlegen einer elektrischen Spannung zur Entsalzung/Probenadsorption erübrigt sich und es müssen auch keine porösen Strukturen hergestellt werden, welche eine Elution der Probe aus dem System (wie z.B. bei den Monolithischen Festphasenxtraktionssystemen) erfordern und das Spektrum der Herstellungs- bzw. Anwendungsmöglichkeiten für die Träger-Chips einschränken.

Diese Vorteile der erfindungsgemäßen Träger-Chips sind nicht allein auf massenspektrometrische Anwendungen beschränkt. Die copolymeren Träger-Chips mit speziellen Oberflächeneigenschaften können auch vorteilhaft für alle anderen Anwendungen eingesetzt werden, in denen die bevorzugte Bindung spezieller Molekültypen an ein Trägermaterial genutzt wird. Beispiele hierfür sind vor allem andere Spektroskopietechniken, z.B. die Emissions- und Absorptionsspektroskopie, und Kombinationen anderer Spektroskopietechniken mit der Massenspektrometrie, z.B. eine Kombination von MALDI-Massenspektrometrie und Fluoreszenzspektroskopie.

In einer speziellen Ausführungsform der vorliegenden Erfindung werden copolymere Oberflächen mit Funktionalitäten verwendet, an die Linker- oder Spacer-Moleküle gekoppelt werden können. An diese können wiederum Moleküle von Interesse in bekannter Weise gebunden werden. Ein Anwendungsbeispiel dieses Prinzip demonstriert der enzymatische Assay von Beispiel 7, worin ein Enzym (alkalische Phosphatase) über ein Spacer-Molekül an die Oberfläche gebunden wurde. Auf diese Weise wurde die freie Beweglichkeit des Enzyms und somit seine Aktivität gegenüber einem ausgewählten Substrat gewährleistet, so dass eine direkte und empfindliche Überwachung der Enzymaktivität mittels MALDI-Massenspektrometrie und UV/VIS-Spektroskopie möglich wurde.

### Figurenbeschreibung

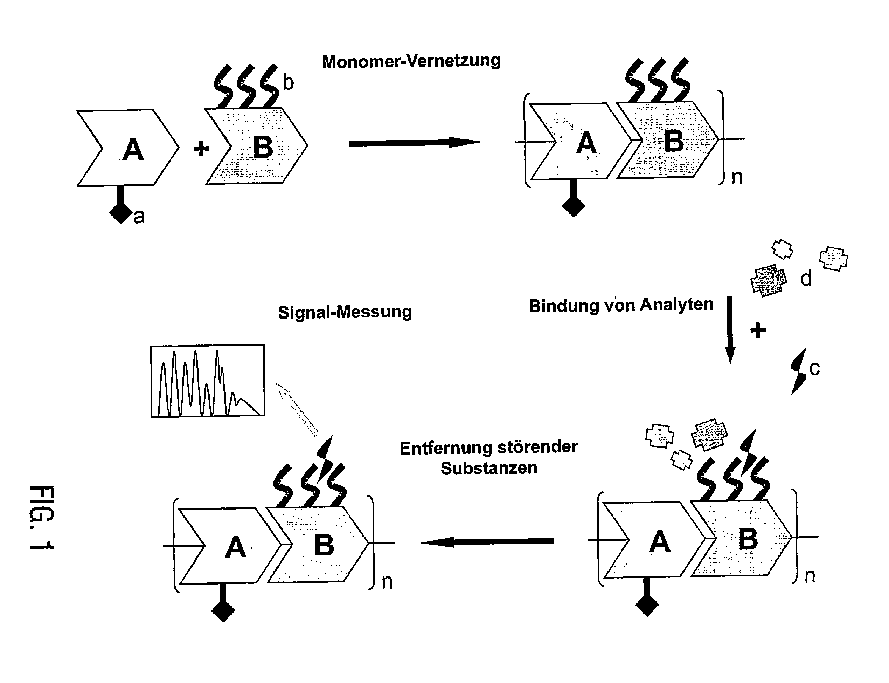

**Fig. 1** ist ein allgemeines Schema, welches die Herstellung von erfindungsgemäßen strukturierten, copolymeren Probenträger-Chips und deren Verwendung in einem spektrometrischen Verfahren illustriert:
   Ausgewählte Monomere (hier A und B) werden unter geeigneten Bedingungen (z.B. Katalysator, UV-Licht) zu einer Kette mit n Einheiten grundsätzlich beliebiger (gewünschtenfalls einstellbarer) Sequenz polymerisiert und stellen auf der Oberfläche des resultierenden Copolymerisats (und durchgehend in dem gesamten Material) unterschiedliche Stellen (a) und (b) für die Wechselwirkung mit den zu untersuchenden Proben bereit. Ein komplexe Probe, die Analyt-Moleküle (c) und störende Moleküle (d) (z.B. Salze, Detergenzien, Puffer etc.) enthält, wird auf die Oberfläche des polymeren Trägerchips aufgebracht. Das Analyt-Molekül bindet aufgrund der speziell eingestellten Oberflächeneigenschaften des Träger-Chips bevorzugt an die Oberfläche und die kontaminierenden Moleküle mit einer niedrigeren Affinität zur Oberfläche werden leicht entfernt. Das von Verunreinigungen befreite Analyt-Molekül liefert dann in dem jeweiligen Nachweissystem ein vergleichsweise stärkeres Signal mit geringerem Hintergrundrauschen.

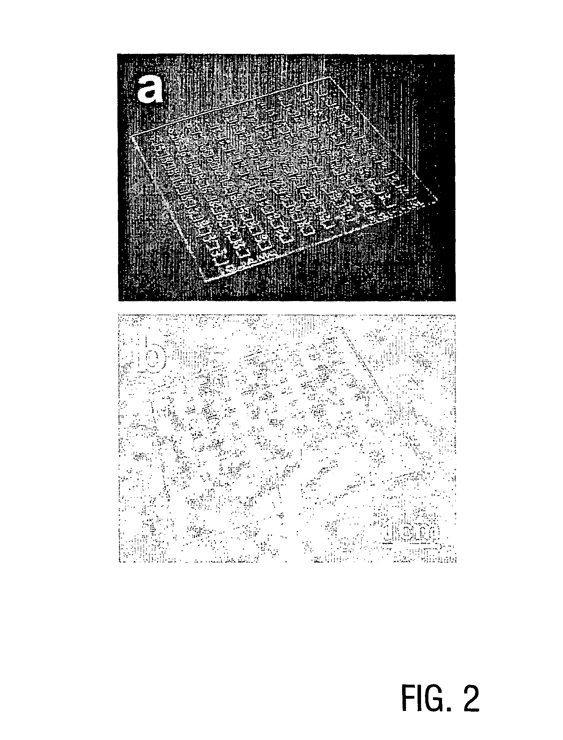

**Fig. 2** zeigt Photographien eines gemäß Beispiel 1 hergestellten Silicium-Wafer-Negativs (a) und des damit gemäß Beispiel 2 hergestellten strukturierten, copolymeren Probenträger-Chips (b).

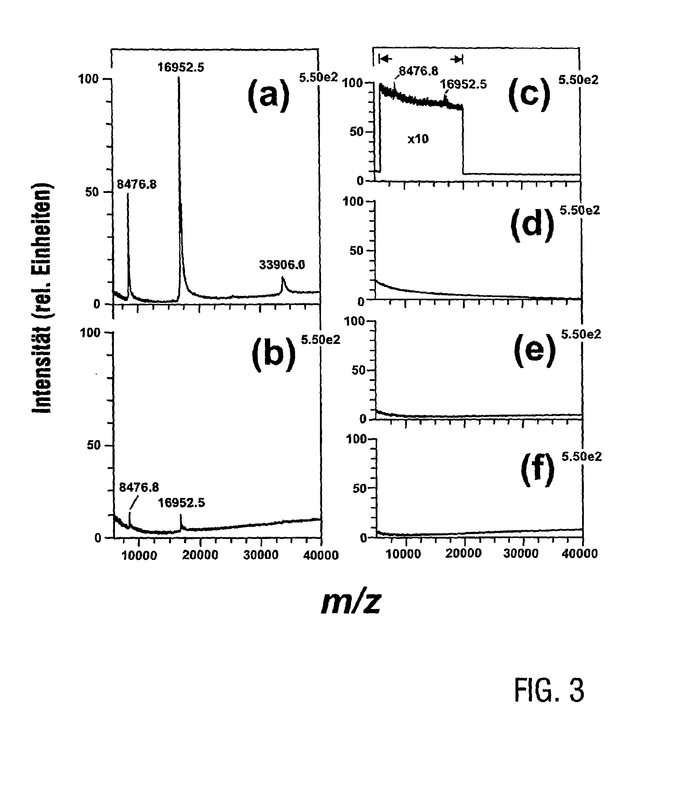

**Fig. 3** zeigt MAZDI-TOF-Spektren von Pferdeherz-Myoglobin in 4 M Harnstoff und 0,1% Trifluoressigsäure auf einem Poly(butylmethacrylat-co-methylmethacrylat)-Probenträger-Chip nach *in situ*-Entsalzung auf dem Träger-Chip (a) und ohne Entsalzung (b) und die entsprechenden Vergleichspektren nach und bevor Entsalzung auf einem PMMA-Probenträger-Chip (c,d) und einem Stahl-Probenträger-Chip (e, f).

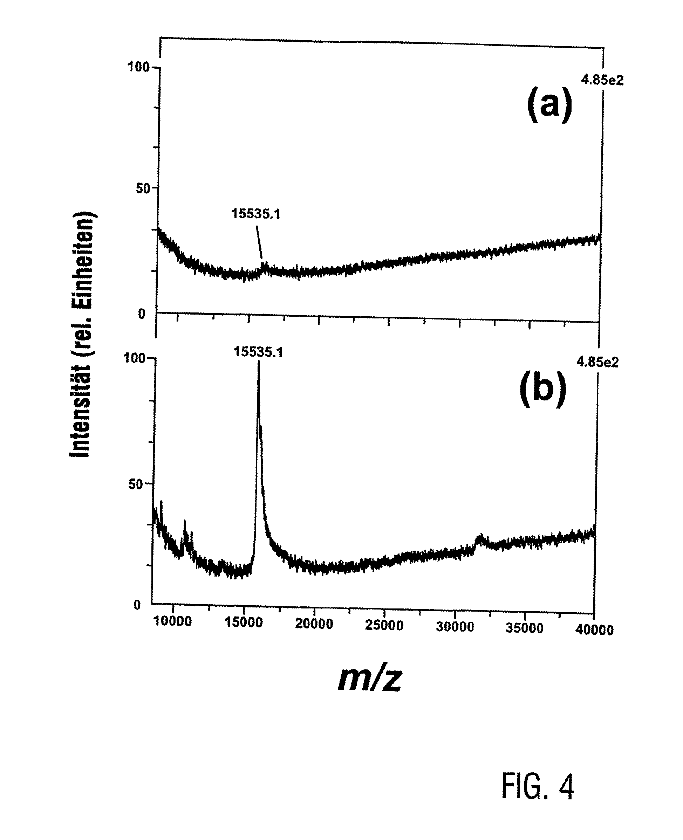

**Fig. 4** zeigt MALDI-TOF-Spektren einer PAGE-Gelelektrophorese-Probe eines Insekten-Δ-11-desaturase-Membranproteinfragments (MG 15534 Da) in 100 mM Phosphat, 10 mM TRIS-Puffer, 0,5 M NaCl-Salz und 10 mM EDTA-Elutionspuffer nach *in situ*-Entsalzung auf dem Poly(butylmethacrylat-co-methyl-methacrylat)-Probenträger-Chip (4b) und Vergleichsspektren ohne Entsalzung (4a).

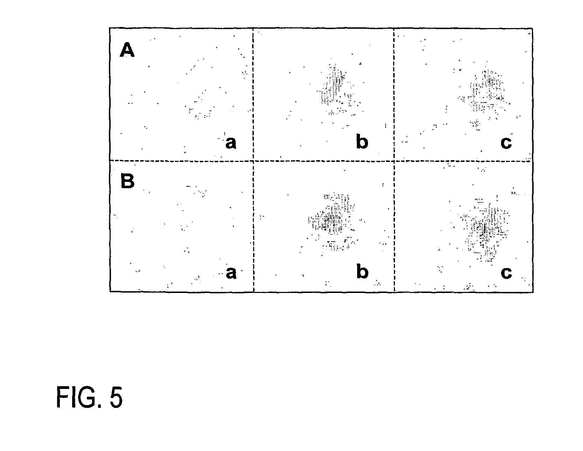

**Fig.5** zeigt Mikrophotographien der enzymatischen Hydrolyse von p-Nitrophenolphosphat (transparent) zu freiem p-Nitrophenol (dunkel) in zwei Parallelansätzen (A, B). Die Felder Aa bzw. Ba entsprechen Mulden, in denen Phosphatase direkt an die Trägeroberfläche gekoppelt wurde, während die Felder Ab, Ac bzw. Bb und Bc Mulden entsprechen, in denen die Phosphatase über einen Linker an den copolymeren Chip gekoppelt war.

Das erfindungsgemäße Verfahren wurde dazu verwendet, um strukturierte Probenträger-Chips für die Massenspektrometrie herzustellen, deren Struktur und Abmessungen denen herkömmlicher Probenträger-Chips aus Stahl entsprach, um die Verwendung in üblichen Massenspektrometriegeräten zu ermöglichen. Konkret wurden Chips in den Abmessungen 50 x 50 x 2 mm mit 100 viereckförmigen Positionen von 2 mm Seitenlänge in 10 x 10 Reihen hergestellt. Der Abstand zwischen den einzelnen Positionen betrug 2,26 mm.

### BEISPIEL 1

Die Herstellung der Chips erfolgte durch Formung unter Atmosphärendruck. Als Negativ wurde zunächst ein Silicium-Wafer vom p-Typ mit <100> Orientierung und 100 mm Durchmesser durch eine Kombination von Photolithographie und nassem chemischem Ätzen hergestellt. Im Gegensatz zu früheren Berichten (Anal. Chem. 2004, 76, 2290-2297) wurde zum Aufbringen der Photolackschicht ein Airbrush-Verfahren verwendet. Eine reproduzierbare Beschichtung von Silicium-Wafern wurde erzielt mit einer selbst hergestellten Glas-Airbrush, bestehend aus einer inneren Sprühvorrichtung (Einlassröhrchen für die Resistlösung von 100 mm x 3,8 mm ID mit einer L-förmigen Sprühdüse von 1 mm Durchmesser) in der Mitte eines äußeren Glasrohrs (20 mm Durchmesser) mit einem vertikalen Lufteinlass (20 mm x 3,8 mm Innendurchmesser) und einem Schliff-Verbindungsstück zu einem 100-ml-Behälter aus braunem Glas. Die Austrittsöffnung der Sprühdüse befand sich zentriert 1 mm von der Austrittsöffnung mit 3,8 mm Durchmesser in dem äußeren Glasrohr. Zur Gewährleistung einer gleichmäßigen Abscheidung von mikroskopischen Tröpfchen des Photoresists wurde der Negativton-Resist SU-8 5 (Microchem, Inc., Newton, MA, USA) mit Aceton verdünnt (50 ml, 3/2 Vol./Vol.). Die Resistmischungen wurden durch zehnminütige Beschallung im Dunkeln homogenisiert und manuell in einer vertikalen Distanz von 20 cm 10 Sekunden lang bei einem konstanten Luftdruck von 2 bar auf den Silicium-Wafer gesprüht. Unter diesen einfachen Bedingungen konnten Resistschichten von 10-15 µm Dicke abgeschieden werden.

Die Photolithographiemasken wurden mit Hilfe einer kommerziellen Bilderzeugungs-Software als Negative der replizierten Strukturen erzeugt und auf Standard-PMMA-Transparentfolien für den Laserdrucker mit einer optimierten Schwarzweiß-Auflösung von 1200 dpi ausgedruckt. Nach der Beschichtung wurden die Wafer 30 Minuten lang bei 90 °C getrocknet und anschließend mit der Photomaske für die UV-Belichtung bedeckt. Die Ausrichtung erfolgte manuell in der "flachen" Orientierung des Wafers. Der Wafer wurde dann mit einer Quarzglasplatte (150 x 150 x 2 mm) bedeckt und 30 Minuten lang UV-Licht ausgesetzt (365 nm, 8 W, 550 µW/cm² in einem Abstand von 15 cm, Carl-Roth). Es folgte eine 40minütige Wärmegradientenbehandlung von 65 bis 90°C (5°C/min.) und anschließend wurde der Wafer 90 s lang in einer Glaspetrischale mit Entwickler (XP SU-8, Microchem, Corp.) entwickelt. Der nicht exponierte Photoresist wurde durch Tränken in 2-Propanol für 30 bzw. 10 s entfernt. Somit wurde nur die gewünschte Struktur auf dem Wafer photopolymerisiert. Anschließend geschah ein "Backen" durch Erwärmung des Wafers für 60 Minuten auf 150°C.

Das nasse chemische Ätzen erfolgte in zwei Schritten. Zuerst wurde die exponierte Siliciumoxidschicht um die mit Photoresist bedeckte Struktur isotrop in einer gepufferten Flußsäurelösung geätzt. Dabei entstand eine SiO₂-Maske, die mit der ursprünglichen Photolithographiemaske identisch war, für den zweiten Schritt der Ätzung des Siliciumsubstrats. Anschließend wurde der Wafer sorgfältig mit destilliertem Wasser gespült und in einer 40%igen KOH-Lösung (enthaltend 5% 2-Propanol) bei 60°C anisostrop unter ständiger Überwachung geätzt, bis die gewünschten Strukturen in der gewünschten Tiefe bzw. Höhe vorlagen (etwa 40 min).

### BEISPIEL 2

Eine offene Form für die Polymerisation wurde gebildet, indem eine quadratische Aluminiumplatte als Spacer zwischen einen wie in Beispiel 1 hergestellten Wafer als Negativ und eine Glasplatte gelegt und mit Teflonband und Laborklemmen befestigt wurde. Der Spacer hatte eine 1 mm breite Einlassöffnung für die Monomerlösungen. Die gewünschten Monomerlösungen (hier Methylmethacrylat und Butylmethacrylat; Verhältnis 4:6, Vol./Vol.) wurden durch Säulenchromatographie mit aktiviertem Aluminiumoxid (Grad CG20) von Hydrochinon-Inhibitor befreit, entgast (z.B. durch Beschallung) und in die Form eingebracht. Die Polymerisationslösung enthielt 0,3 % (Gew./Vol.) Bezoinmethylether als Katalysator. Die Polymerisation erfolgte durch UV-Bestrahlung ((365 nm, 8 W, Carl Roth) für etwa 1 Stunde in einer belüfteten Abzugshaube. Die Polymerisate wurden durch Beschallung (10 min. in Wasser bei 40°C) aus der Form befreit und in 2-Propanol gereinigt. Zur Eliminierung innerer Spannungen wurden die Polymerisate einer 10minütigen Wärmebehandlung bei etwa 70°C in einen Konvektionsofen unterworfen. Nach langsamem Abkühlen auf Umgebungstemperatur wurden die Träger über Nacht im Vakuum gelagert, um etwaige restliche Spuren von Monomeren zu entfernen. Eine Inspektion mit einem optischen Mikroskop zeigte eine hohe Replikationsgenauigkeit der gebildeten Strukturen ohne Verzerrungen oder Schrumpfung.

### BEISPIEL 3

Ein wie in Beispiel 2 hergestellter Probenträger-Chip aus Poly(butylmethacrylat-co-methylmethacrylat)(Verhältnis MMA: BMA 4:6, Vol./Vol.), ein auf dieselbe Weise hergestellter Probenträger-Chip aus Polymethylmethacrylat-Homopolymer und ein herkömmlicher Probenträger-Chip aus Stahl wurden zur massenspektrometrischen Analyse von Pferdeherz-Myoglobin (90 %, Sigma-Aldrich) eingesetzt. Die Polymerträger-Chips wurden ohne vorherige Oberflächenbehandlung verwendet, während die Probenträger-Chips aus rostfreiem Stahl zur Entfernung etwaiger Verunreinigungen mit konzentrierter Salpetersäure unter kurzer Beschallung gewaschen, mit deionisiertem Wasser abgespült und an Luft getrocknet wurden. 1,2 µl Myoglobinlösung (in 0,1%iger Trifluoressigsäure, TFA) wurden mit 1,2 µl einer Lösung von Sinapinsäure-Matrix (9 mg/ml in 60/40 Vol./Vol. 0,1 % TFA/Acetonitril) mit Hilfe von 10-µl-Eppendorfspitzen gemischt und Aliquots von jeweils 1,2 µl auf die Auftragspositionen aufgebracht. Die Tropfen wurden an der Luft bei Umgebungstemperatur trocknen gelassen und die Träger-Chips in die MALDI-Quelle eingeführt. Die Proben, welche Puffersalze enthielten, wurden auf dieselbe Weise hergestellt und bei Umgebungstemperatur getrocknet. Die Entsalzungsprozedur wurde durch einfache Aufbringung von 1,5 µl 0,1%iger TFA für 1 Minute, gefolgt von Tröpfchenentfernung mit Hilfe einer neuen 10-µl-Pipettenspitze, durchgeführt. Die entsalzten Auftragspositionen wurden trocknen gelassen und in die MALDI-Quelle eingeführt.

Die Spektren wurden unter Verwendung eines TofSpec 2E-MALDI TOF-Instruments (Micromass, Manchester, UK), das sowohl in einem Linear- als auch Reflectron-Modus mit verzögerter Extraktion betrieben wurde, aufgezeichnet. Die Desorption/- Ionisation erfolgte mit einem CO₂-UV-Laser (337 nm, 4-ns-Pulse mit 180 mJ). Die positiven Ionen wurden einem Beschleunigungspotential von 20 kV ausgesetzt und mit einem dualen Mikrokanalplatten (MCP)-Detektor nachgewiesen. Matrixionen wurden mit einem Ausschluss niedriger Massen (m/z 600) unterdrückt. Es wurden jeweils 5 Laserimpulse pro Messung ausgelöst und die erhaltenen Spektren waren Mittelwerte von mindestens 20 aufeinanderfolgenden Messungen. Geglättete und grundlinien-korrigierte Daten wurden erforderlichenfalls unter Verwendung berechneter mono-isotopischer Massen von (M+H]-Peaks kalibriert. Die Daten wurden mit Hilfe der Mass-Lynx 3.2-Software auf einer PC-Arbeitsstation aufgezeichnet und analysiert.

Fig. 3a zeigt das Spektrum einer Myoglobinprobe von 10 pmol in 4 M Harnstoff, welche wie oben beschrieben mit Matrixlösung gemischt und aufgebracht worden war. Nach Trocknen und Entsalzen durch einminütige Inkubation mit 0,1%iger TFA-Lösung waren die vorwiegend in der wässerigen Phase gelösten Salze entfernt und die hydrophoberen Proteine auf dem hydrophoben Probenträger-Chip zurückgehalten. Die kleine Menge an Matrix, welche ebenfalls verblieb, war ausreichend, um das Protein zu ionisieren. Es wurde ein starker Molekülionen-Peak neben dem doppelt protonierten Molekülion und einfach protonierten Dimer-Peaks beobachtet.

Eine analoge Probenvorbehandlung auf einer PMMA-Oberfläche resultierte in einem erheblich geringeren Grad an Proteinadsorption, was eine niedrigere Bindungstendenz für den untersuchten Proteintyp anzeigt, und ergab nur relativ schwache Signale von Myoglobin (Fig. 3c), während mit dem Vergleichsprobenträger-Chip aus Stahl keinerlei Signal beobachtet wurde (Fig. 3e).

Die Messung einer nicht-entsalzten Probe auf dem neuen butylmodifizierten Probenträger-Chip ergab ebenfalls noch nachweisbare Signale (3b), während keine Signale auf der PMMA-Oberfläche (3d) oder dem Stahlträger-Chip (3f) ohne Entsalzen beobachtet wurden.

### BEISPIEL 4

Eine MALDI-TOF-Massenspektrometrie unter den in Beispiel 3 beschriebenen Bedingungen wurde mit 20 pmol des Δ-11-Desaturase-Membranproteinfragments 90-180 (MG 15534 Da) in einem komplexen Puffer, enthaltend 100 mM Natriumdihydrogenphosphat und 10 mm TRIS-Puffer, 0,5 M NaCl und 10 mM EDTA, durchführt. In einem Ansatz wurde die Proben wie in Beispiel 3 beschrieben entsalzt und die effiziente Adsorption des hydrophoben Membranproteins an den erfindungsgemäßen Probenträger-Chip resultierte in einem intensiven Molekülionen-Peak (Fig. 4b), während die nicht entsalzte Probe eines Vergleichsansatzes nur ein minimales Signal ergab (Fig. 4a).

### BEISPIEL 5

Probenträger-Chips aus Poly(methylmethacrylat-co-2-sulfoethylmethacrylat) (Material A) bzw. Poly(methylmethacrylat-comethacrylat) (Material B) wurden als Repräsentanten von Copolymeren mit negativ geladenen Funktionalitäten in einer Form wie in Beispiel 2 beschrieben hergestellt.

Zunächst wurde eine Inhibitor-freie Lösung des Methylmethacrylat-Monomers (Polysciences Inc., Warrington, PA) mit 2-Sulfoethylmethacrylat (Polysciences Inc.) in einem Verhältnis von 97:3 (Vol./Vol.) oder mit Methacrylsäure (Polysciences Inc.) in einem Verhältnis von 94:6 (Vol./Vol.) gemischt. Dann wurde jeweils 0.3 % (Gew./Vol.) Benzoinmethylether (Polysciences Inc.) als UV-Katalysator zugegeben und die Lösungen wurden 1 h lang UV-Licht mit 365 nm Wellenlänge ausgesetzt, um eine viskose Präpolymer-Lösung zu erhalten. Eine solche Präpolymer-Lösung kann gewünschtenfalls über längere Zeiträume gekühlt aufbewahrt werden. Vor dem Formen wurden die viskosen Lösungen 1 Minute lang im Vakuum gehalten, um zu verhindern, dass während des Polymerisationsvorgangs Gas freigesetzt würde. Die Lösungen wurde dann in identischen Formen mit der gewünschten negativen Struktur eingebracht und etwa 2 h lang unter UV-Licht mit einer Wellenlänge von 365 nm polymerisiert. Die Befreiung aus der Form erfolgte wie in Beispiel 2 beschrieben. Nach dem Spülen der Oberflächen der hergestellten Probenträger mit 5 mM Ammoniumhydroxidlösung waren die Oberflächen infolge der Dissoziation von Protonen der Carboxy- bzw. Sulfofunktionalitäten negativ geladen.

1 µl eines tryptischen Phosphorylase B-Verdaus (MassPrep, Waters Inc.), entsprechend 100 fmol eines jeden Peptids, in 50 mM Ammoniumhydrogencarbonatpuffer, pH 8,5, wurde auf die jeweiligen Trägeroberflächen aufgebracht, um die Adsorptionseffizienz zu testen. Nach 1 Minute Inkubation wurden die Lösungen mit Hilfe einer 10-µl-Pipettenspitze entfernt und 1 µl α-Cyano-4-hydroxyzimtsäure-Matrix (5 mg/ml in Ethanol/Acetonitril/0.2 % Trifluoressigsäure 6/3,5/0,5 Vol./Vol.) aufgebracht. Nach dem Trocknen wurden die Träger-Chips in die MALDI-Ionenquelle eingeführt und Massenspektren wurden in einem Positivionen-Reflektron-Modus aufgezeichnet.

Es wurden eine Reihe intensiver Peaks von einfach protonierten Peptiden im m/z-Bereich zwischen 800-2000 Da beobachtet. Die intensivsten Peaks wurden als [M+H]⁺-Peaks von T92 (SwissProt Database) bei 1278,67 Dalton, gefolgt von T86 bei 1053,65 Da und T70 bei 869,29 Da identifiziert. Dies demonstrierte die Adsorption von positiv geladenen Peptiden an die Copolymer-Zieloberflächen. Zur Beurteilung der Effizienz der Puffersalzentfernung und der Nachweisempfindlichkeit für die Materialien A und B wurde das mittlere Signal-RauschVerhältnis (S/R) für Signale berechnet, die über 1 Minute Aufzeichnung gemittelt worden waren. Das mit Sulfoethylmethacrylat modifizierte Copolymer A ergab ein S/R-Verhältnis von 45 für den Peak von 1278,67 Da gegenüber einem S/R-Verhältnis von 2 für eine Probe ohne Entsalzung. Ein höherer S/R-Wert von 62 wurde für das identische Ion für Copolymer B (Methacrylat-modifiziertes Material) gegenüber einem S/R-Wert von 2,5 für die nicht entsalzte Probe erhalten. Der leicht niedrigere Wert für Material A dürfte auf eine partielle Aufnahme von Wassermolekülen aufgrund der erhöhten Hydrophilizität des Materials zurückzuführen sein.

### BEISPIEL 6

Zum Testen der Probenvorbehandlung und Signalverbesserung bei der MALDI-TOF-Analyse komplexer Nukleinsäureproben wurde ein positiv geladener Copolymer-Träger-Chip unter Verwendung einer Mischung von Methylmethacrylat und Aminoethylmethacrylat in einem Verhältnis von 98:2(Vol./Vol.) hergestellt.

Das 2-Aminoethylmethacrylat wurde zunächst nach dem folgenden Verfahren extrahiert: 2-Aminoethylmethacrylat-Hydrochlorid (Polysciences Inc., Warrington, PA, USA) wurde in Wasser (0,7 g in 10 ml entionisiertem Wasser) gelöst, die Säure wurde durch Zugabe von Natriumcarbonat (Sigma) (0,7 g in 50 ml Wasser) und 20minütiges Schütteln neutralisiert. Das freie Monomer wurde mit Ethylacetat (Sigma) extrahiert (3 x 40 ml) und das verbleibende Carbonat durch Ausschütteln mit einer gesättigten Lösung von NaCl (Sigma) (10 ml) entfernt. Die organische Phase wurde abgetrennt und im Vakuum eingedampft. Die Ausbeute bei der Extraktion betrug etwa 20 %. Die viskose Aminoethyl-Monomerlösung wurde mit Inhibitor-freier Methylmethacrylat-Lösung gemischt und die Mischung wurde analog zu Beispiel 5 zunächst unter UV-Licht in eine Präpolymer-Lösung überführt und dann unter UV-Licht polymerisiert. Die Copolymerisate wurden wie bereits beschrieben aus der Form gelöst und über Nacht unter Vakuum aufbewahrt.

2 µl einer DNA-Leiter (enthaltend 10, 20 und 30 bp, entsprechend etwa 3500, 7000 und 14000 Da, Promega) in einer Konzentration von 60 pmol/µl in 10 mM Tris-HCl-Puffer, pH 4,9, 50 mM KCl, 5 mM MgCl₂ und 0,1 mM EDTA wurden auf den Probenträger-Chip aufgebracht,um die Signalverbesserung zu testen. Zum Vergleich wurde ein Standard-Stahlprobenträger-Chip (Micromass, UK) verwendet. Die Lösungen wurden an Luft getrocknet und 1 Minute lang mit 2 µl entionisiertem Wasser gespült, wobei eine 10-µl-Pipettenspitze verwendet wurde. Dann wurden 4 µl 3-Hydroxypicolinsäure-Matrix (60 mg/ml), gelöst in einer Mischung von Acetonitril/Wasser mit einem relativ niedrigen Acetonitrilanteil(3/7) auf die Zielgebiete aufgebracht. Nach dem Trocknen wurden die Probenträger-Chips in die MALDI-Ionenquelle eingeführt und Massenspektren im Negativionen-Modus aufgezeichnet.

Negativmodus-Spektren von entsalzten DNAs auf den aminhaltigen Copolymer-Trägeranordnungen waren durch intensive Peaks der beiden kleineren Oligomeren von 10 und 20 bp und einen schwächeren Peak der 30-bp-DNA charakterisiert. Im Vergleich dazu waren die Signale, welche für Proben ohne den Spülschritt und für Proben auf dem Stahlprobenträger aufgezeichnet worden waren, gegenüber dem Hintergrund nicht aufgelöst. Dies kann auf eine bevorzugte Retention von negativ geladenen DNA-Phosphatgruppen durch die protonierten Aminfunktionalitäten während der Salzentfernung und die Elimierung verbleibender K⁺- und Na⁺-Ionen (welche zu einer Peak-Verbreiterung bei der herkömmlichen MALDI-MS mit einem Stahlprobenträger-Chip führen) während der Ionisierung durch Bindung an einige deprotonierte Carboxylgruppen zurückgeführt werden.

Die obigen Beispiele belegen die erheblichen Vorteile, die mit dem Einsatz von erfindungsgemäßen copolymeren Träger-Chips als Probenträger-Chips für Biomoleküle, wie z.B. Proteine und Nukleinsäuren, insbesondere in massenspektrometrischen Assays erzielt werden können.

### BEISPIEL 7

Zum Testen der Eignung von copolymeren Probenträger-Chips als Plattformen für die Durchführung enzymatischer Reaktionen und für deren schnellen Nachweis wurde ein t-Butylcarboxyaminoethylmethacrylat/Methylmethacrylat-Copolymer-Chip (in einem Vol./Vol.-Verhältnis von 1:9 aus Monomeren polymerisiert) hergestellt. Somit trugen Abschnitte der Polymerkette alkylierte Aminofunktionalitäten, welche schnell zu freien Aminogruppen aktiviert werden konnten, um ein Enzym über ein Spacer-Molekül zu binden, womit die freie Beweglichkeit des Enzyms und somit seine Aktiviät mit einem ausgewählten Substrat sichergestellt wurde, um eine chemische Umwandlung des Substrats zu veranlassen, welche eine direkte Überwachung der Enzymaktivitäten mittels MALDI-Massenspektrometrie und UV/VIS-Spektroskopie ermöglichte.

Die Oberfläche eines t-Butylcarboxyaminoethylmethacrylat/- Methylmethacrylat-Copolymer-Chips wurde mit 2 µl einer Mischung von HCl. konz./MeOH (1:1 Vol./Vol.) aktiviert. Die Oberfläche des Chips wurde mit reichlich Wasser gespült und 5 Minuten lang in einem Ultraschallbad in bidest. Wasser gehalten. 2 µl 0,1 M NaHCO₃-Puffer, pH 9, wurden in die 2x2 mm große Probenzone eingebracht, um etwaige Säurerückstande auf der Oberfläche zu neutralisieren. Nach wiederholtem Spülen und 5-minütiger Behandlung in einem Ultraschallbad wurde der Chip in einem Stickstoffstrom getrocknet und 2 µL N-hydroxysulfosuccinimid-(polyethylenglycol)-Biotin (NHS-PEO₄-Biotin-Vernetzungssonde von Pierce Inc., Rockford, USA) in 0,1 M Phosphatpuffer, pH 7,4, wurden in die Probenmulde eingebracht. Die Probenmulde wurde 1 h lang in einem Feuchtluft-Laborinkubator bei 15 °C und leichter mechanischer Bewegung mit 300 UpM inkubiert. Die Oberfläche der Mulde wurde dann mit bidest. Wasser gespült und 2 µl einer Blockierungslösung (5mg/ml Rinderserumalbumin in 0,1 M Phosphat/Borat-Puffer, pH 7,4) wurden in die aktivierte Mulde eingebracht, um etwaige unspezifische Wechselwirkungen von Proteinen mit der Oberfläche zu vermeiden. Die Blockierungslösung wurde in der Mulde 1 h lang bei 15 °C und leichter mechanischer Bewegung mit 300 UpM in der Feuchtluft-Inkubationskammer inkubiert. Die Muldenoberfläche wurde mit reichlich Wasser gespült, in einem Stickstoffstrom getrocknet und mit 2 µl von 10 E/ml alkalischer Phosphatase, vernetzt mit Streptavidin (Sigma), überschichtet. Das Enzym wurde 1 h lang bei 15 °C und leichter mechanischer Bewegung mit 300 UpM in einem Feuchtluft-Laborinkubator inkubiert. Die Chip-Oberfläche wurde mit Wasser gespült. Schließlich wurden 2 µl p-Nitrophenylphosphat (0,25 M) in 0,1 M TRIS-Puffer, pH 7,4, der Probenmulde zugegeben.

Eine schnelle enzymkatalysierte Hydrolyse wurde mit einer UVVIS-Spektralphotometer-Kammer beobachtet, die auf 570 nm eingestellt war. Eine Extinktionsänderung von 0,001 auf einen Maximalwert von 0,09 wurde innerhalb von 2,6 Min. beobachtet. Fig. 5 zeigt Mikrophotographien der enzymatischen Hydrolyse von p-Nitrophenylphosphat (transparent) zu freiem p-Nitrophenol (dunkel) in zwei parallelen Versuchen (A, B in Fig. 5). Referenzmulden mit Enzym, das direkt an die Copolymer-Oberfläche adsorbiert worden war, zeigten eine geringere Farbänderung nach Zugabe von Substrat und Inkubation, wohingegen Mulden, die alkalische Phosphatase über den Linker mit dem polymeren Chip verknüpft enthielten, eine hohe Hydrolyserate zeigten (Ab, Ac bzw. Bb, Bc in Fig. 5).

Die Aktivität des Enzyms wurde anschließend in der Quelle des MALDI-TOF-Spektrometers überprüft. Die Chip-Oberfläche wurde an Luft getrocknet und die Mulden (enthaltend alkalische Phosphatase und deren Substrat p-Nitrophenolphosphat) wurden mit 0,6 µl Hydroxypikolinsäure-Matrix (60 mg/ml in 7/3 Wasser/Acetonitril) überschichtet. Nach Einführung des Kunststoff-Chips in die MALDI-Quelle und Etablierung des Vakuums wurden deprotonierte Einzelladungs-Peaks von p-Nitrophenylphosphat im Negativ-Reflektron-Modus gemessen. Die Aktivität der enzymatischen Reaktion wurde durch Integration der Molekülpeakflächen von p-Nitrophenylphosphat gemessen. Im Vergleich zu den Mulden der Referenzexperimente (Fig. 5, Aa und Ba), worin nur eine etwa 2-5%ige Abnahme der p-Nitrophenylphosphatkonzentration beobachtet wurde, zeigten die Mulden, die alle Systemkomponenten (aktivierte copolymere Oberfläche, Linker, hydrolytisches Enzym und Substrat) enthielten, eine 88-92%ige Abnahme der Peakfläche, was eine vollständig quantitative Hydrolyse des Substrats auf dem copolymeren Chip anzeigt.

## Patentansprüche

1. Strukturierter Probenträger-Chip für die Spektrometrie oder Spektroskopie, bestehend aus einem Copolymer aus Methylmethacrylat und mindestens einem weiteren Monomer, das stärker hydrophob als Methylmethacrylat ist, oder mindestens einem weiteren Monomer, bei dem es sich um ein substituiertes Alkylmethacrylat, das eine Sulfo-, Hydroxy- oder Carboxyfunktionalität oder eine Aminofunktionalität aufweist, handelt.

2. Probenträger-Chip nach Anspruch 1, **dadurch gekennzeichnet, dass** das weitere Monomer aus einem der Alkylmethacrylate Ethylmethycrylat bis Octadecylmethacrylat ausgewählt ist.

3. Probenträger-Chip nach Anspruch 2, **dadurch gekennzeichnet, dass** das weitere Monomer Butylmethacrylat ist.

4. Probenträger-Chip nach Anspruch 1, **dadurch gekennzeichnet, dass** das weitere Monomer 2-Sulfoethylmethacrylat, 2-Aminoethylmethacrylat oder t-Butylcarboxyaminoethylmethacrylat ist.

5. Probenträger-Chip nach einem der Ansprüche 1-4, **dadurch gekennzeichnet, dass** der Träger planar ist und die Struktur eine Tiefe im Bereich von 1 bis 1000 µm aufweist.

6. Probenträger-Chip nach einem der Ansprüche 1-5, **dadurch gekennzeichnet, dass** es sich um einen Träger für die Massenspektrometrie, insbesondere MALDI- oder SELDI-Massenspektrometrie, handelt.

7. Verwendung des strukturierten Probenträger-Chips nach einem der Ansprüche 1-6 in einem massenspektrometrischen Assay zum Nachweis von Biomolekülen und Metaboliten, einschließlich von Proteinen, Peptiden, Nukleinsäuren und niedermolekularen Substanzen.

8. Verwendung des strukturierten Probenträger-Chips nach einem der Ansprüche 1-5 in spektroskopischen Verfahren.

9. Verwendung nach Anspruch 8 in der Emissions- oder Absorptionsspektroskopie.

10. Verwendung des strukturierten Probenträger-Chips nach einem der Ansprüche 1-6 in einem Verfahren bei dem Massenspektrometrie und spektroskopische Verfahren kombiniert werden.

11. Verwendung nach Anspruch 10, bei der MALDI-Massenspektrometrie und Emissions- und/oder Absorptionsspektroskopie kombiniert werden.

12. Verwendung nach Anspruch 11, bei der MALDI-Massenspektrometrie und Fluoreszenzspektroskopie kombiniert werden.

## Claims

1. A structured sample support chip for use in spectrometry or spectroscopy, consisting of a copolymer from methylmethacrylate and at least one other monomer that is more hydrophobic than methylmethacrylate or at least one other monomer that is a substituted alkylmethacrylate comprising a sulfo, hydroxy or carboxy functionality or an amino functionality.

2. The sample support chip acccording to Claim 1, **characterized in that** the other monomer is selected from the alkylmethacrylates ethylmethacrylate through octadecylmethacrylate.

3. The sample support chip acccording to Claim 2, **characterized in that** the other monomer is butylmethacrylate.

4. The sample support chip acccording to Claim 1, **characterized in that** the other monomer is 2-sulfoethylmethacrylate, 2-aminoethylmethacrylate or t-butylcarboxyaminoethylmethacrylate.

5. The sample support chip according to any one of Claims 1-4, **characterized in that** the support is planar and that the structure has a depth in a range of 1 to 1000 µm.

6. The sample support chip acccording to any one of Claims 1-5, **characterized in that** it is a support for use in mass spectrometry, in particular MALDI- or SELDI mass spectrometry.

7. Use of the structured sample support chip according to any one of Claims 1-6 in a mass spectrometric assay for the detection of biomolecules and metabolites, including proteins, peptides, nucleic acids and lower-molecular substances.

8. The use of the structured sample support chip according to any one of Claims 1-5 in spectroscopic methods.

9. The use according to Claim 8 in emission or absorption spectroscopy.

10. The use of the structured sample support chip according to any one of Claims 1-6 in a method in which mass spectrometry and spectroscopic methods are combined.

11. The use according to Claim 10, in which MALDI mass spectrometry and emission and/or absorption spectroscopy are combined.

12. The use according to Claim 11, in which MALDI mass spectrometry and fluorescence spectroscopy are combined.

## Revendications

1. Puce support d'échantillon structuré pour la spectrométrie ou la spectroscopie, constituée d'un copolymère de méthacrylate de méthyle et d'au moins un autre monomère, qui est plus fortement hydrophobe que le méthacrylate de méthyle, ou d'au moins un autre monomère, qui est un méthacrylate d'alkyle substitué, qui présente une fonctionnalité sulfo, hydroxy ou carboxy ou une fonctionnalité amino.

2. Puce support d'échantillon selon la revendication 1, **caractérisée en ce que** l'autre monomère est choisi parmi un des méthacrylates d'alkyle méthacrylate d'éthyle à méthacrylate d'octadécyle.

3. Puce support d'échantillon selon la revendication 2, **caractérisée en ce que** l'autre monomère est le méthacrylate de butyle.

4. Puce support d'échantillon selon la revendication 1, **caractérisée en ce que** l'autre monomère est le méthacrylate de 2-sulfoéthyle, le méthacrylate de 2-aminoéthyle ou le méthacrylate de t-butylcarboxyaminoéthyle.

5. Puce support d'échantillon selon l'une des revendications 1 à 5, **caractérisée en ce que** le support est plane et la structure présente un creux sur une profondeur de 1 à 1000 µm.

6. Puce support d'échantillon selon l'une des revendications 1 à 5, **caractérisée en ce que** c'est un support pour la spectrométrie de masse, en particulier la spectrométrie de masse MALDI ou SELDI.

7. Utilisation de la puce support d'échantillon structuré selon l'une des revendications 1 à 6 dans une analyse de spectrométrie de masse pour détecter des biomolécules et des métabolites, y compris des protéines, des peptides, des acides nucléiques et des substances de faibles poids moléculaires.

8. Utilisation de la puce support d'échantillon structuré selon l'une des revendications 1 à 5 dans des procédés spectroscopiques.

9. Utilisation selon la revendication 9 dans la spectroscopie d'émission ou d'absorption.

10. Utilisation de la puce support d'échantillon structuré selon l'une des revendications 1 à 6 dans un procédé dans lequel la spectrométrie de masse et les procédés spectroscopiques sont combinés.

11. Utilisation selon la revendication 10, dans laquelle la spectrométrie de masse MALDI et la spectroscopie d'émission et/ou d'absorption sont combinées.

12. Utilisation selon la revendication 11, dans laquelle la spectrométrie de masse MALDI et la spectroscopie de fluorescence sont combinées.
